# EUROPÄISCHE PATENTSCHRIFT

(11) **EP 0 328 911 B1**
(45) Veröffentlichungstag und Bekanntmachung des Hinweises auf die Patenterteilung: **20.07.1994**
(21) Anmeldenummer: 89101323.7
(22) Anmeldetag: 26.01.1989
(51) Int. Cl.: A61C 8/00, A61C 1/08

(54) **Bohrvorrichtung für kieferchirurgische Implantatkavitäten**
Drilling device for implant cavities of the jawbone
Dispositif de perçage pour cavités d'implants chirurgicaux des mâchoires

(30) Priorität: 29.01.1988 DE 3802789
(43) Veröffentlichungstag der Anmeldung: 23.08.1989
(73) Patentinhaber: Lauks, Nikola, Dr., D-22397 Hamburg (DE)
(72) Erfinder: Lauks, Nikola, Dr., D-22397 Hamburg (DE)
(74) Vertreter: Gosch, Wolf-Dietrich

(56) Entgegenhaltungen:
- DE-U- 1 945 653
- DE-U- 8 625 288
- US-A- 3 407 503
- US-A- 3 508 334

## Beschreibung

Die Erfindung betrifft eine Bohrvorrichtung für kieferchirurgische Implantatkavitäten mit einem einen rotierenden Bohrer aufnehmenden Winkelstück.

Die für die Implantation eines Zylinderimplantates erforderliche Bohrung im Kieferknochen eines Patienten weist vielfältige Probleme auf. In einigen Fällen muß auf Implantate verzichtet werden, da keine anatomischen Voraussetzungen bestehen, d. h. es besteht keine ausreichende Knochensubstanz für eine Implantatkavität. Üblicherweise werden derartige Bohrungen freihändig in den Kieferknochen eingebracht. Hierbei kann es leicht zu Fehlbohrungen kommen. Besonders groß ist die Gefahr, zu tief zu bohren, so daß der Sinus Maxillaris oder der Canalis Mandibularis trepaniert werden. In gleicher Weise besteht die Gefahr, schief zu bohren und damit die Spongiosa in transversaler Dimension stark zu verletzen. Durch diese Verletzung ist eine Knochenresorption vorprogrammiert. Diese transversale Dimension muß besondere Betrachtung finden, da bei einer Implantation mindestens eine 1mm breite Spongiosa zwischen einem Implantat und der Lamina externa und interna des jeweiligen Kieferkammes verbleiben muß, damit eine ausreichende Durchblutung der Implantatsumgebung gewährleistet ist. Eine Nichtbeachtung dieser Dimension ist verantwortlich für viele Mißerfolge. Dabei ist zu berücksichtigen, daß besonders später, wenn die Implantate belastet werden und wenn sie nicht exakt parallel zueinander stehen - Implantat zu Implantat und/oder Implantat zu Zahn -, eine vertikotransversale Druckübertragung auf das Implantat hervorgerufen wird. Durch diese vertikotransversale Belastung entsteht auf der einen Seite des Implantates ein Druckeffekt und auf der gegenüberliegenden Seite ein Zugeffekt. Beide Effekte bewirken auf längere Zeit eine trichterförmige Knochenresorption, welche für eine Sekundärinfektion der Implantatumgebung Voraussetzungen schafft.

Aus der US-A-3 407 503 ist eine Vorrichtung zum Einbringen von Bohrungen in Zähne bekannt, bis welcher parallelverlaufende Bohrbuchsen an einer Bohrbuchsenschablone befestigt sind. Zum Einbringen von Bohrungen in den Zahn wird der von Winkelhandstück aufgenommene Bohrer durch die jeweilige Bohrbuchse geführt.

Eine ähnliche Vorrichtung ist aus der DE-U-1 945 653 bekannt.

Aufgabe der vorliegenden Erfindung ist es daher, eine Bohrvorrichtung der eingangs genannten Art zu schaffen, mit deren Hilfe Implantationskavitäten auf engem Raum geschaffen werden können und die Richtung der Kavitäten exakt vorherbestimmt werden können.

Diese Aufgabe wird erfindungsgemäß durch die Vorrichtung gemäß Anspruch 1 gelöst. Damit kann eine dreidimensionale Vorbereitung einer Implantatkavität sowie eine exakte Lokalisation derselben ermöglicht werden. Eine Gefahr des Verlustes der Spongiosa oder einer Verletzung des Patienten wird zuverlässig vermieden. So können je nach anatomischen Möglichkeiten mehrere Implantate parallel zueinander implantiert werden und alle die erforderlichen dreidimensionalen Voraussetzungen erfülleb. Durch die Parallelität und die Möglichkeit einer exakten topografischen Vorplanung der Implantatkavität erreicht man eine Fülle von prothetischen Versorgungsmöglichkeiten, die nach dem gegenwärtigen Entwicklungsstand noch auf eine Brücke oder eine Stegprothese begrenzt sind. Wegen der mangelnden Parallelität der Implantate kommt es zu vertiko - transversalen Belastungen, wodurch der Steg oder die Brücke traumatisiert werden. Dies äußert sich dann beispielsweise darin, daß sich die Implantatschraube in kurzer Zeit lockert.

Die Geschiebebohrbuchse ist erfindungsgemäß mit einem das Hand- bzw. Winkelstück führenden Geschiebe versehen. Hierdurch ist es möglich, den Bohrer vom ersten Ansetzen auf dem Kieferknochen des Patienten bis zur endgültigen Bohrtiefe exakt zu führen und ein Abweichen von der einmal vorgewählten Bohrrichtung zuverlässig zu vermeiden.

Voraussetzung für eine oder mehrere optimale Implantatkavitäten ist es, eine Bohrbuchsenschablone herzustellen, um die Geschiebebohrbuchsen ausreichend sicher in bezug auf den zu behandeln den Kiefer festzulegen.

Mit Hilfe des Schablonenkörpers, der nach dem Aufmodellieren auf das Modell und nach seinem Aushärten mit Geschiebebohrbuchsen versehen wird, die nach den Verhältnissen des Patienten optimal ausgerichtet und mit dem Schablonenkörper zuverlässig verbunden werden können, wird die Bohrbuchsenschablone hergestellt.

Weitere Einzelheiten der Erfindung ergeben sich aus der nachfolgenden ausführlichen Beschreibung und den beigefügten Zeichnungen, in denen eine Ausführungsform der erfindungsgemäßen Vorrichtung beispielsweise veranschaulicht ist.

In den Zeichnungen zeigen:

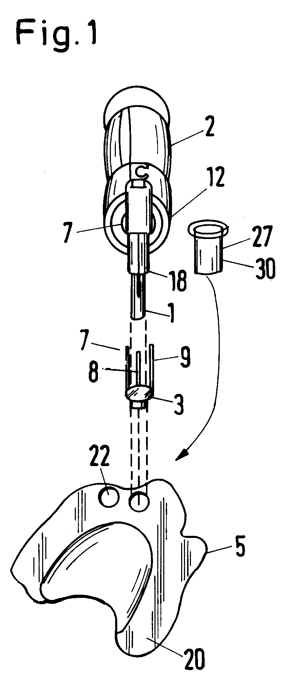

- Fig. 1 :: Eine perspektivische Darstellung eines mit einem Bohrer versehenen Winkelstückes einer Geschiebebohrbuchse, einer Präzisionshülse und einem Schablonenkörper;

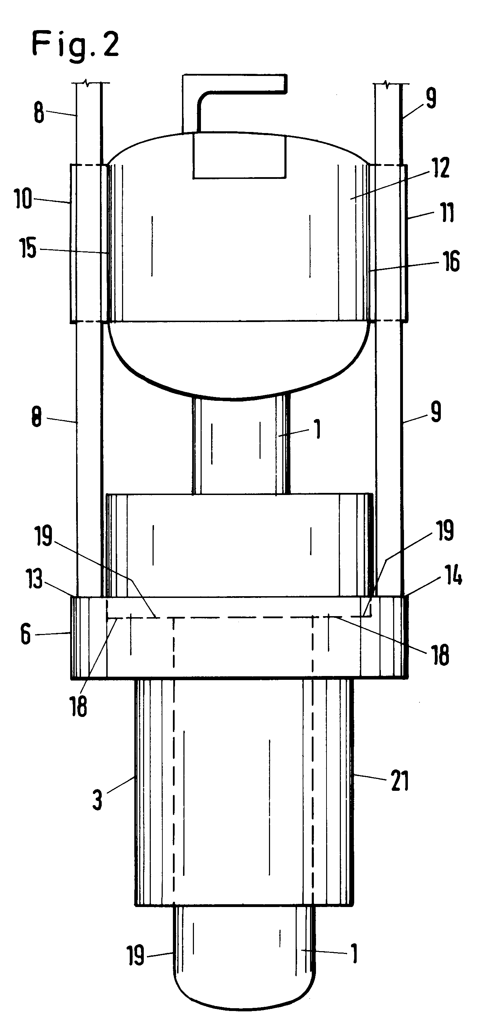

- Fig. 2 :: eine Vorderansicht eines Winkelstückes mit eingesetztem Bohrer, der durch eine Geschiebebohrbuchse hindurchragt;

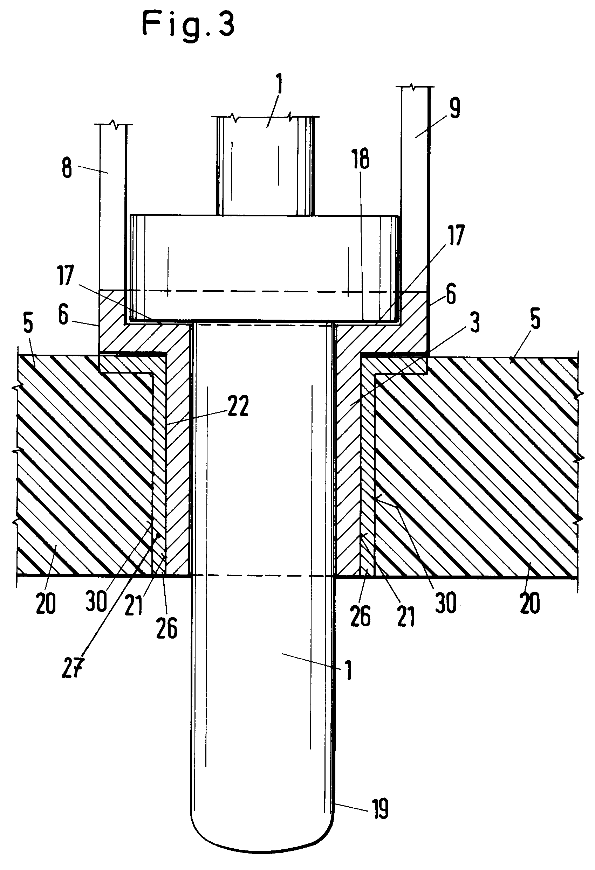

- Fig. 3 :: einen Schnitt durch eine Bohrbuchsenschablone mit eingesetzter Geschiebebohrbuchse und durch diese hindurchragenden Bohrer;

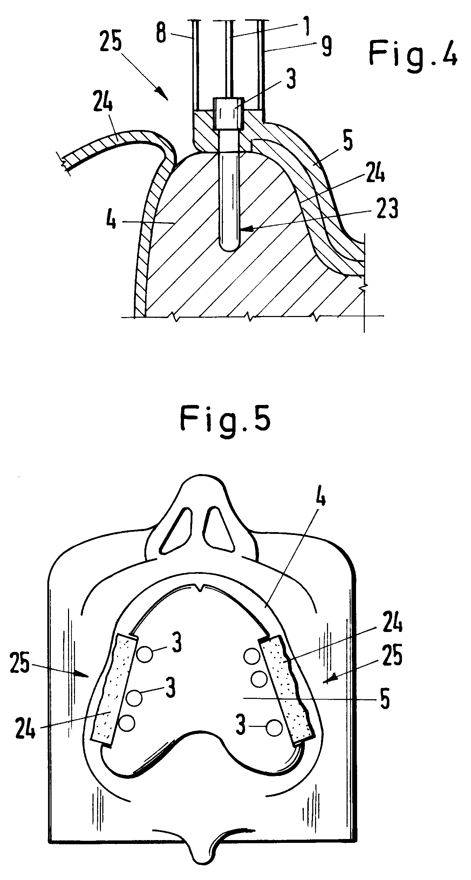

- Fig. 4 :: einen Querschnitt durch einen Kiefer mit einer aufgelegten Bohrbuchsenschablone mit eingesetzter Geschiebebohrbuchse und durch diese hindurchragenden Bohrer;
- Fig. 5 :: eine Druntersicht unter eine auf einen Oberkiefer aufgelegte Bohrbuchsenschablone;

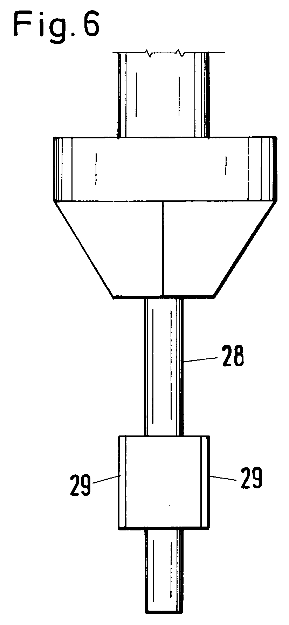

- Fig. 6 :: eine Seitenansicht eines Spannkopfes des Parallelometers mit einem Führungsstift für eine Geschiebebohrbuchse.

Eine erfindungsgemäße Bohrvorrichtung für kieferchirurgische Implantatkavitäten besteht im wesentlichen aus einem einen rotierenden Bohrer 1 aufnehmenden Winkelstück 2 und einer Geschiebebohrbuchse 3, durch die der Bohrer hindurchragt und die an einer dem Kiefer 4 des zu behandelnden Patienten angepaßten Bohrbuchsenschablone 5 gehaltert ist.

Die Geschiebebohrbuchse 3 ist im wesentlichen zylindrisch geformt und weist einen Innendurchmesser auf, der einen Außendurchmesser des Bohrers 1 übersteigt. Die Geschiebebohrbuchse 3 ist an ihrer dem Winkelstück 2 zugewandten Seite mit einem sie radial umgebenden Ringflansch 6 versehen. Sie weist ein das Winkelstück 2 führendes Geschiebe 7 auf. Das Geschiebe 7 verläuft einer Rotationsachse des Bohrers 1 parallel. Das Geschiebe 7 besteht aus zwei Führungsstegen 8, 9, die auf zwei am Winkelstück 2 angeordneten U-Profilen 10, 11 gleitend gelagert sind. Die Führungsstege 8, 9 sind an dem Ringflansch 6 befestigt, den sie parallel zur Rotationsachse des Bohrers 1 überragen. Die U-Profile 10, 11 sind am Winkelstück benachbart einer den Bohrer 1 aufnehmenden Fassung 12 befestigt. Vorzugsweise sind die Führungsstege 8, 9 an einander gegenüberliegenden Bereichen 13, 14 des Ringflansches 6 befestigt, während die beiden U-Profile 10, 11 einander parallel laufen und an einander gegenüberliegenden Seitenflächen 15, 16 der Fassung 12 angeordnet sind.

Die Geschiebebohrbuchse 3 ist im Bereich des Ringflansches 6 mit einer ringförmigen Auflagerfläche 17 versehen, an der sich ein den Bohrer 1 umgebender Tiefenanschlag 18 bei Erreichen der vorbestimmten Bohrtiefe abstützt. Um eine Mehrzahl verschiedener Bohrtiefen sicherstellen zu können, reicht es aus, eine entsprechende Anzahl von Bohrern 1 vorzuhalten, deren spanabhebende Bohrkronen 19 die Tiefenanschläge 18 um jeweils vorgewählte Maße überragen. Ebenso können die Maße des Ringflansches 6 und des Abstandes zwischen der Unterfläche 26 der Geschiebebohrbuchse 3 und der Auflagerfläche 17 das Maß der Bohrtiefe bestimmen.

Die Geschiebebohrbuchse 3 ist auswechselbar an der Bohrbuchsenschablone 5 befestigt. Diese besteht aus einer Kunststoffplatte, die einen Schablonenkörper 20 ausbildet, der einem Gipsabdruck eines Kiefers 4 eines zu behandelnden Patienten angepaßt ist. Die Bohrbuchsenschablone 5 weist eine Materialstärke auf, die im wesentlichen der Höhe eines zylindrischen Bereiches 21 der Geschiebebohrbuchse 3 angepaßt ist. Die Bohrbuchsenschablone 5 ist von zylindrischen Ausnehmungen 22 durchdrungen, in denen Präzisionshülsen 27 befestigt sind, in denen die Geschiebebohrbuchsen 3 mit ihren zylindrischen Bereichen 21 einander parallel verlaufend gehaltert sind. Diese Halterung wird im wesentlichen durch die zylindrischen Innenflächen der Präzisionshülsen 27 gegenüber den zylindrischen Bereichen 21 der Geschiebebohrbuchsen 3 herbeigeführt. Die zylindrischen Bereiche 21 sind gegenüber den Präzisionshülsen 27 in ihren Durchmessern in einer Weise angepaßt, daß zwischen den zylindrischen Bereichen 21 und den Präzisionshülsen 27 eine eine präzise Halterung bewirkende Haftreibung besteht.

Gemäß einer weiteren Ausführungsform der Erfindung ist es möglich, das Geschiebe 7 mit lediglich einem Führungssteg 8 auszubilden, der auf einem U-Profil 10 gleitend gelagert ist. Vorzugsweise sind in diesem Falle der Führungssteg 8 und das U-Profil 10 formschlüssig im Sinne der Verhinderung möglicher Drehbewegungen aneinander angepaßt. Es ist aber auch möglich, das Geschiebe 7 aus drei Führungsstegen 8, 9 und drei U-Profilen 10, 11 zu bilden. Anstelle von U-Profilen 10, 11 ist es auch möglich, geschlossene Hülsen über die Führungsstege 8, 9 gleiten zu lassen. Schließlich ist es möglich, ein Geschiebe 7 aus einer Metallhülse zu bilden, deren Innendurchmesser einem zylindrischen Außendurchmesser der den Bohrer 1 aufnehmenden Fassung 12 angepaßt ist.

Das Verfahren zur Herstellung einer Bohrbuchsenschablone 5 kann wie folgt durchgeführt werden:
Die restlichen Zähne eines Patienen werden im Sinne einer Parallelität klinischer Kronen präpariert. Dann wird ein anatomischer Abdruck genommen, ein Modell ausgegossen. In einem Parallelometer werden die Zähne auf ihre Parallelität geprüft. Ist diese erreicht, wird auf dem Modell einer aus plastischem, aushärtbarem Kunststoff bestehender Schablonenkörper 20 aufmodelliert. Dieser Schablonenkörper ist so gestaltet, daß er beim Aufklappen des Zahnfleisches und des submukosen Gewebes 24 nicht störend ist. Der Schablonenkörper 20 muß also für das Operationsfeld 25 formiert sein.

Wenn der Schablonenkörper 20 ausgehärtet ist, wird an einer Stelle, an der später am Patienten die Implantatkavität 23 gebohrt wird, eine Bohrung durch den Schablonenkörper 20 in das Modell durchgeführt. Dafür wird das Modell mit einem Parallelometertisch in ein Fräsgerät eingesetzt, so daß die Bohrung zu den restlichen Zähnen oder übrigen Implantatkavitäten 23 parallel verläuft.

Nach dieser Bohrung, die entsprechend der vorgesehenen Stärke des Implantates einen Durchmesser von 4 oder 3,3 mm hat, kann man am Modell kontrollieren, wie breit die Spongiosasubstanz vom Implantat zur Lamina externa und zur Lamina interna beim Patienten sein wird. Besteht an der vorgesehenen Stelle, an der die Implantatkavität 23 entstehen soll, eine Ostose, wird das Modell geglättet, wie dies auch später am Patienten erfolgt, so daß die in die im Schablonenkörper vorgesehene Geschiebebohrbuchse 3 ein breiteres Bohrplateau erhält. Ist dieses erfolgt, wird das vorgebohrte Loch im Schablonenkörper 20 bis auf einen Durchmesser von ca. 7 mm erweitert, so daß eine in ihren dem Schablonenkörper 20 zugekehrten Bereichen 30 vorgesehene Präzisionshülse 27 mit einem Durchmesser von etwa 6 mm einen genügenden Spielraum besitzt, um gegebenenfalls versatzt zu werden, falls der Abstand der Bohrkavität im Modell zur Lamina externa und zur Lamina interna falsch gewählt worden ist. Die Präzisionshülse 27 wird in folgender Weise fixiert: Zuerst wird das Modell samt dem Schablonenkörper 20 und dem Parallelometertisch in den Parallelometer zurückgestellt. Am Parallelometer wird statt des Parallelometerstiftes ein Führungsstift 28 mit U-Profilen 29 eingespannt, die in ihrer Form und Ausbildung den U-Profilen 10, 11 am Winkelstück 2 entsprechen und die mit den Führungsstegen 8, 9 der Geschiebebohrbuchse 3 ein Geschiebe ausbilden. Auf den Führungsstift 28 wird eine Geschiebebohrbuchse 3 mit aufgesteckter Präzisionshülse 27 aufgesteckt und in das im Durchmesser größere vorgebohrte Loch in dem Schablonenkörper 20 und dem Modell geführt, bis die Präzisionshülse 27 mit ihrer Unterfläche 26 auf der Modelloberfläche aufliegt. Nunmehr kann gegebenenfalls eine unrichtige Lage der Präzisionshülse 27 korrigiert und diese in optimaler Lage festgelegt werden. Danach wird die Präzisionshülse 27 mit dem Schablonenkörper 20 mit Simplexkunststoff in ihrer endgültigen Lage fest verbunden. Nach der Autopolymerisation des Kunststoffes kann die Geschiebebohrbuchse 3 aus der Präzisionshälse herausgezogen werden und bei Bedarf wieder eingesteckt werden.

Entsprechend der vorgewählten Zahl von Implantatkavitäten 23 kann dieser Vorgang wiederholt werden, so daß nach Einsetzen sämtlicher Geschiebebohrbuchsen 3 die Bohrbuchsenschablone 5 fertiggestellt ist. Es ist auch möglich, lediglich eine Geschiebebohrbuchse 3 zu verwenden, die nacheinander in mehreren Präzisionshülsen 27 eingesteckt wird.

Die Bohrbuchsenschablone 5 wird auf den Kiefer 4 des zu behandelnden Patienten aufgelegt, nach dem das Operationsfeld 25 vorbereitet wurde, indem das Zahnfleisch und Submukosagewebe 24 von dem Kiefer 4 abgeklappt worden ist. Durch Verwendung von mehreren Bohrern 1 mit ansteigenden Durchmessern und einer einzigen Geschiebebohrbuchse 3 ist es möglich, Implantatkavitäten 23 in den Kiefer 4 einzubringen, ohne den Patienten einem zusätzlichen Verletzungsrisiko auszusetzen. Über die Geschiebebohrbuchse 3 bzw. das Geschiebe 7 wird das Winkelstück 2 und der mit ihm verbundene Bohrer 1 jeweils exakt so geführt, wie dies am Modell und mit Hilfe der Röntgenbilder zuvor berechnet worden ist.

Die Erfahrung mit vorbeschriebenem Verfahren hat gezeigt, daß es sogar möglich ist, die Operation durchzuführen, ohne das Zahnfleisch und das Submukosagewebe abzuklappen. Die Bohrung wird durch das Zahnfleisch und Submokusgewebe hindurchgeführt. Dadurch werden Schmerz und Infektionsgefahr weiter erheblich verringert.

## Patentansprüche

1. Bohrvorrichtung für kieferchirurgische Implantatkavitäten mit einem einen rotierenden Bohrer (1) aufnehmenden Winkelstück, mit einer Geschiebebohrbuchse (3) durch die der Bohrer (1) hindurchragt, die an einer dem Kiefer (4) eines zu behandelnden Patienten angepaßten Bohrbuchsenschablone (5) gehaltert ist, und die mit einem das Winkelstück (2) führenden Geschiebe (7) versehen ist.

2. Bohrvorrichtung nach Anspruch 1, wobei die Geschiebebohrbuchse (3) im wesentlichen zylindrisch geformt ist und einen Innendurchmesser aufweist, der einen Außendurchmesser des Bohrers (1) übersteigt.

3. Bohrvorrichtung nach Anspruch 1 und 2, wobei die Geschiebebohrbuchse (3) an ihrer dem Winkelstück (2) zugewandten Seite einen sie radial umgebenden Ringflansch (6) aufweist.

4. Bohrvorrichtung nach Anspruch 1 bis 3, wobei das Geschiebe (7) einer Rotationsachse des Bohrers (1) parallel verläuft.

5. Bohrvorrichtung nach Anspruch 1 bis 4, wobei das Geschiebe (7) aus mindestens einem Führungssteg (8, 9) besteht, der auf einem U-Profil oder einer geschlossenen Hülse (10, 11) gleitend gelagert ist.

6. Bohrvorrichtung nach Anspruch 1 bis 5, wobei der Führungssteg (8, 9) an dem Ringflansch (6) befestigt ist, den er parallel zur Rotationsachse des Bohrers (1) überragt.

7. Bohrvorrichtung nach Anspruch 1 bis 6, wobei das U-Profil (10, 11) am Winkelstück (2) benachbart einer den Bohrer (1) aufnehmenden Fassung (12) befestigt ist.

8. Bohrvorrichtung nach Anspruch 1 bis 7, wobei das Geschiebe (7) aus jeweils zwei Führungsstegen (8, 9), die an einander gegenüberliegenden Bereichen (13, 14) des Ringflansches (6) befestigt sind und zwei U-Profilen (10, 11) besteht, die einander parallel verlaufen und an einander gegenüberliegenden Seitenflächen (15, 16) der Fassung (12) vorgesehen sind.

9. Bohrvorrichtung nach Anspruch 1 bis 7, wobei das Geschiebe (7) aus je drei Führungsstegen (8, 9) und drei U-Profilen (10, 11) besteht.

10. Bohrvorrichtung nach Anspruch 1 bis 3, wobei das Geschiebe (7) aus einer Metallhülse besteht, deren Innendurchmesser einem zylindrischen Außendurchmesser der Fassung (12) angepaßt ist.

11. Bohrvorrichtung nach Anspruch 1 bis 10, wobei die Geschiebebohrbuchse (3) im Bereich des Ringflansches (6) eine ringförmige Auflagerfläche (17) aufweist, an der sich ein den Bohrer (1) an seinem Schaft umgebender Tiefenanschlag (18) abstützt.

12. Bohrvorrichtung nach Anspruch 1 bis 11, wobei eine Mehrzahl von Bohrern (1) mit unterschiedlich langen Bohrkronen (19) bezogen auf den Tiefenanschlag (18) vorgesehen sind.

13. Bohrvorrichtung nach Anspruch 1 bis 12, wobei die Geschiebebohrbuchse (3) auswechselbar an der Bohrbuchsenschablone (5) befestigt ist.

14. Bohrvorrichtung nach Anspruch 1 bis 13, wobei die Bohrbuchsenschablone (5) aus einem aus Kunststoff bestehenden Schablonenkörper (20) besteht, der einem Gipsmodell angepaßt ist, das einem Kiefer (4) eines zu behandelnden Patienten identisch nachgebildet ist.

15. Bohrvorrichtung nach Anspruch 1 bis 14, wobei der Schablonenkörper (20) eine Materialstärke aufweist, die im wesentlichen der Höhe eines zylindrischen Bereiches (21) der Geschiebebohrbuchse (3) angepaßt ist.

16. Bohrvorrichtung nach Anspruch 1 bis 15, wobei die Bohrbuchsenschablone (5) von zylindrischen Ausnehmungen (22) durchdrungen ist, in denen die Geschiebebohrbuchsen (3) einander parallel verlaufend gehaltert sind.

17. Bohrvorrichtung nach Anspruch 1 bis 16, wobei in den Ausnehmungen (22) Präzisionshülsen (27) befestigt sind, deren Innendurchmesser einem Außendurchmesser des zylindrischen Bereiches (21) der Geschiebebohrbuchse (3) im Sinne einer Haftreibung angepaßt ist.

## Claims

1. A drilling apparatus for implant cavities of jaw surgery, having an elbow piece which receives a rotating drill (1), having a sliding drill bush (3) through which the drill (1) projects and which is mounted on a drill bush template (5) matched to the jaw (4) of a patient to be treated, and which is provided with a sliding mechanism (7) which guides the elbow piece (2).

2. A drilling apparatus according to claim 1, wherein the sliding drill bush (3) is shaped substantially cylindrically and has an inside diameter which exceeds an outside diameter of the drill (1).

3. A drilling apparatus according to claims 1 and 2, wherein the sliding drill bush (3) has an annular flange (6) radially surrounding it on its side facing the elbow piece (2).

4. A drilling apparatus according to claims 1 to 3, wherein the sliding mechanism (7) runs parallel to an axis of rotation of the drill (1).

5. A drilling apparatus according to claims 1 to 4, wherein the sliding mechanism (7) consists of at least one guide projection (8, 9) which is slidably mounted on a U-shaped section or on a closed sleeve (10, 11).

6. A drilling apparatus according to claims 1 to 5, wherein the guide projection (8, 9) is attached to the annular flange (6), beyond which it projects parallel to the axis of rotation of the drill (1).

7. A drilling apparatus according to claims 1 to 6, wherein the U-shaped section (10, 11) is attached to the elbow piece (2) adjacent to a mounting (12) which receives the drill (1).

8. A drilling apparatus according to claims 1 to 7, wherein the sliding mechanism (7) consists in each case of two guide projections (8, 9) which are attached to opposite regions (13, 14) of the annular flange (6), and of two U-shaped sections (10, 11) which run parallel to each other and which are provided on opposite side faces (15, 16) of the mounting (12).

9. A drilling apparatus according to claims 1 to 7, wherein the sliding mechanism (7) consists in each case of three guide projections (8, 9) and three U-shaped sections (10, 11).

10. A drilling apparatus according to claims 1 to 3, wherein the sliding mechanism (7) consists of a metal sleeve, the inside diameter of which is matched to a cylindrical outside diameter of the mounting (12).

11. A drilling apparatus according to claims 1 to 10, wherein the sliding drill bush (3) has an annular support face (17) in the region of the annular flange (6), on which support face a bit stop (18) which surrounds the shank of the drill (1) is supported.

12. A drilling apparatus according to claims 1 to 11, wherein a plurality of drills (1) is provided, having drill bits (19) of different lengths in relation to the bit stop (18).

13. A drilling apparatus according to claims 1 to 12, wherein the sliding drill bush (3) is interchangeably attached to the drill bush template (5).

14. A drilling apparatus according to claims 1 to 13, wherein the drill bush template (5) consists of a template body (20) consisting of plastic, which is matched to a plaster model which is an identical copy of a jaw (4) of a patient to be treated.

15. A drilling apparatus according to claims 1 to 14, wherein the template body (20) comprises a thickness of material which is substantially matched to the height of a cylindrical region (21) of the sliding drill bush (3).

16. A drilling apparatus according to claims 1 to 15, wherein the drill bush template (5) is penetrated throughout by cylindrical recesses (22) in which the sliding drill bushes (3) are mounted running parallel to each other.

17. A drilling apparatus according to claims 1 to 16, wherein precision bushings (27) are fastened in the recesses (22), the inside diameters of which precision bushings are matched to an outside diameter of the cylindrical region (21) of the sliding drill bush (3) in the sense of a frictional bond.

## Revendications

1. Dispositif de perçage pour des activités d'implants de la chirurgie de le mâchoire comportant une pièce coudée contenant un perçoir rotatif (1), comportant un guide de perçage à galet (3) traversé par le perçoir (1), qui repose sur un gabarit de guide de perçage (5) adapté à la mâchoire (4) d'un patient à traiter, et qui est muni d'un galet (7) guidant la pièce coudée (2).

2. Dispositif de perçage selon la revendication 1, dans lequel le guide de perçage à galet (3) est essentiellement de forme cylindrique et présente un diamètre intérieur qui dépasse un diamètre extérieur du perçoir (1).

3. Dispositif de perçage selon les revendications 1 et 2, dans lequel le guide de perçage à galet (3) présente une bride annulaire (6) l'entourant radialement sur son côté faisant face à la pièce coudée (2).

4. Dispositif de perçage selon les revendications 1 à 3, dans lequel le galet (7) suit parallèlement un axe de rotation du perçoir (1).

5. Dispositif de perçage selon les revendications 1 à 4, dans lequel le galet (7) comprend au moins une nervure de guidage (8, 9) qui glisse sur un profilé en U ou une douille fermée (10, 11).

6. Dispositif de perçage selon les revendications 1 à 5, dans lequel la nervure de guidage (8, 9) est fixée à la bride annulaire (6), qu'il surplombe parallèlement à l'axe de rotation du perçoir (1).

7. Dispositif de perçage selon les revendications 1 à 6, dans lequel le profilé en U (10, 11) est fixé à la pièce coudée (2) adjacente à une monture (12) contenant le perçoir (1).

8. Dispositif de perçage selon les revendications 1 à 7, dans lequel le galet (7) se compose respectivement de deux nervures de guidage (8, 9), qui sont fixées à des parties opposées (13, 14) de la bride annulaire (6), et de deux profilés en U (10, 11), qui sont parallèles l'un par rapport à l'autre et se trouvent sur des faces latérales opposées (15, 16) de la monture (12).

9. Dispositif de perçage selon les revendications 1 à 7, dans lequel le galet (7) comprend respectivement trois nervures de guidage (8, 9) et trois profilés en U (10, 11).

10. Dispositif de perçage selon les revendications 1 à 3, dans lequel le galet (7) se compose d'une douille métallique, dont le diamètre intérieur est adapté à un diamètre extérieur cylindrique de la monture (12).

11. Dispositif de perçage selon les revendications 1 à 10, dans lequel le guide de perçage à galet (3) présente, dans la zone de la bride annulaire (6), une surface portante annulaire (17), sur laquelle repose une butée de profondeur (18) entourant le perçoir (1) sur sa tige.

12. Dispositif de perçage selon les revendications 1 à 11, dans lequel est prévue une pluralité de perçoirs (1) avec des couronnes de perçage de longueurs différentes (19) par rapport à la butée de profondeur (18).

13. Dispositif de perçage selon les revendications 1 à 12, dans lequel le guide de Perçage à galet (3) est fixé de manière amovible au gabarit de guide de perçage (5).

14. Dispositif de perçage selon les revendications 1 à 13, dans lequel le gabarit de guide de perçage (5) est composé d'un corps de gabarit (20) en plastique, qui correspond à une maquette en plâtre, reconstituée de manière identique d'après la mâchoire (4) d'un patient à traiter.

15. Dispositif de perçage selon les revendications 1 à 14, dans lequel le corps de gabarit (20) présente une épaisseur de matériau qui correspond pour l'essentiel à la hauteur d'une partie cylindrique (21) du guide de perçage à galet (3).

16. Dispositif de perçage selon les revendications 1 à 15, dans lequel le gabarit de guide de perçage (5) est percé par des creux cylindriques (22), dans lesquels les guides de perçage à galet (3) sont maintenus parallèlement les uns par rapport aux autres.

17. Dispositif de perçage selon les revendications 1 à 16, dans lequel des douilles de précision (27) sont fixées à l'intérieur des creux (22), dont le diamètre intérieur est adapté à un diamètre extérieur de la partie cylindrique (21) du guide de perçage à galet (3) dans le sens d'un frottement par adhérence.
